(19) Europäisches Patentamt
European Patent Office
Office européen des brevets

(11) Numéro de publication: **0 199 661**
**B1**

(12) # FASCICULE DE BREVET EUROPEEN

(45) Date de publication du fascicule du brevet: **31.05.89**

(51) Int. Cl.⁴: **C07C 45/46**, C07C 45/76, C07C 49/782

(21) Numéro de dépôt: **86420094.4**

(22) Date de dépôt: **27.03.86**

(54) **Procédé de préparation d'acylbiphényles.**

(30) Priorité: **16.04.85 FR 8505909**

(43) Date de publication de la demande: **29.10.86 Bulletin 86/44**

(45) Mention de la délivrance du brevet: **31.05.89 Bulletin 89/22**

(84) Etats contractants désignés: **AT BE CH DE FR GB IT LI LU NL SE**

(56) Documents cités:
**EP-A- 0 026 991**
**EP-A- 0 069 598**
**GB-A- 2 030 158**
**US-A- 4 276 226**

**CHEMICAL ABSTRACTS, vol. 101, no. 5, juillet 1984, page 486, résumé no. 38218u, Columbus, Ohio, US**
**JOURNAL OF POLYMER SCIENCE: POLYMER CHEMISTRY EDITION, vol. 17, no. 7, juillet 1979, pages 2337-2350, John Wiley & Sons, Inc.; SHIOW-CHING LIN et al.: "New processable polyaromatic ether-keto-sulfones curable by entramolecular cyclization. XV"**
**Jouranl of Medicinal Chemistry 1973, vol. 16, no. 11, page 1207**

(73) Titulaire: **RHONE-POULENC CHIMIE, 25, quai Paul Doumer, F-92408 Courbevoie Cédex(FR)**

(72) Inventeur: **Crochemore, Michel, Domaine de Gilbertin 3, rue des Lilas, F-69630 - Chaponost(FR)**

(74) Mandataire: **Le Pennec, Magali et al, RHONE-POULENC INTERSERVICES Service Brevets Chimie 25, Quai Paul Doumer, F-92408 Courbevoie Cédex(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 199 661 B1**

ACTORUM AG

## Description

La présente invention a pour objet un procédé de préparation d'acylbiphényles par acylation de biphényles et plus particulièrement la préparation de diacyl-4-4' biphényles par acylation du biphényle.

Les diacylbiphényles sont des produits industriels utilisés comme intermédiaires en synthèse organique. Par oxydation par l'eau oxygénée en présence d'un acide ils conduisent aux dérivés hydroxylés correspondants conformément à la réaction de BAEYER et VILLIGER. Par amination réductrice ils peuvent être transformés en amines au moyen du mélange ammoniac/hydrogène (cf. brevet américain 2.763.688). Il est ainsi possible d'obtenir à partir des diacylbiphényles des composés difonctionnels tels que les dihydroxybiphényles ou les diamines à motifs biphényles utilisables pour la préparation de polycondensats.

On a proposé divers procédés d'acylation du biphényle et de ses dérivés de substitution. Le procédé le plus couramment utilisé consiste à faire réagir un biphényle avec un agent d'acylation tel qu'un anhydride d'acide, un halogénure d'acide ou un cétène, en présence de chlorure d'aluminium, dans un solvant tel que le sulfure de carbone ou le chlorure de méthylène. Les rendements en produits de mono-et/ou diacylation sont variables selon les conditions de la réaction, cf. notamment N.L. DRAKE et al J. Am. Chem. Soc. 52 page 3715 et suivantes (1930); S.L. SILVER et al J. Am. Chem. Soc. 56 pages 2429–2431 (1934); J.W. WILLIAMS et al J. Am. Chem. Soc. 61 pages 3438–3429 (1939); L.M. Mong et al J. Am. Chem. Soc. 63 pages 1939–1940 (1941); H.C.BROWN et al J. Am. Chem. Soc. 84 pages 1236–1238 (1962); Chemical Abstracts, vol. 101, n° 5, p. 486; n° 38218u, Journal of Polymer Science, vol. 17, p. 2337–2350; brevet américain n° 2 763 688. L'inconvénient majeur de ce procédé réside dans la nécessité de recourir à l'emploi de quantités considérables de chlorure d'aluminium (au moins égales à une mole par mole de biphényle) ce qui limite fortement son intérêt industriel. On a encore suggéré de procéder à l'acétylation du biphényle au moyen de l'acide acétique dans l'acide fluorhydrique anhydre qui joue à la fois le rôle de catalyseur de Friedel et Crafts et de milieu réactionnel (cf. W.F. HUBER et al J. Am. Chem. Soc. 68 pages 1109–1112 [1946]). Dans ces conditions le rendement en diacétyl 4-4'-biphényle est nul malgré le recours à des températures de l'ordre de 80 à 110°C et à des pressions de 6 à 8 bars. Il s'avère donc qu'aucun des procédés d'acylation du biphényle proposés à ce jour ne s'avère pleinement satisfaisant du point de vue industriel. La présente invention a pour objet un procédé de préparation de diacyl-4-' biphényles par acylation des biphényles exempt des inconvénients des procédés de l'art antérieur.

Les brevets EP 26991, US 4276226, GB 2030158 décrivent l'acylation de phénols ou de dérivés phénoliques fortement activés vis à vis de la substitution. Ces composés peuvent subir l'acylation en présence de HF seul, de $BF_3$ seul mais ne nécessitent pas la présence concommitante de HF et de $BF_3$.

Le brevet EP 69598 décrit l'acylation d'halogénobenzènes en présence du couple catalytique HF et $BF_3$. L'ensemble des dérivés subissant l'acylation présente une constante de Hammet, indiquant la désactivation du composé, nettement inférieure à celle de l'acéthylbiphényle, composé obtenu à l'issue de la première acylation.

Il n'était pas évident que l'acéthylbiphényle puisse subir la deuxième acylation.

Un premier objet de la présente invention réside dans un procédé de diacylation de biphényles conduisant à des taux de transformation élevés des biphényles et à des rendements élevés en diacylbiphényles.

Un autre objet de la présente invention réside dans un procédé d'acylation de biphényles pouvant être conduit dans des conditions douces de température et de pression.

Un autre objet de la présente invention réside dans un procédé d'acylation de biphényles permettant la récupération simple du catalyseur et des divers composants du milieu réactionnel.

Plus spécifiquement la présente invention a pour objet un procédé de préparation de diacyl-4-4' biphényle caractérisé en ce qu'on met en contact à une température supérieure ou égale à 50°C le biphényle et un agent acylant dans l'acide fluorhydrique liquide en présence de trifluorure de bore en quantité telle que la pression absolue de trifluorure de bore dans l'enceinte réactionnelle soit d'au moins un bar...

On a constaté qu'en réalisant l'acétylation du biphényle dans l'acide fluorhydrique anhydre en présence de trifluorure de bore on obtient des rendements élevés en diacétylbiphényle et un taux de transformation complet du biphényle.

Les agents acylants qui conviennent à la mise en œuvre du procédé selon l'invention sont ceux habituellement utilisés dans les réactions de Friedel et Crafts (cf. G.A. OLAH, Friedel-Crafts and related Reactions volume III partie 1, Intersciense Publishers 1964). On peut donc faire appel à des acides carboxyliques, leurs anhydrides, leurs esters ou leurs halogénures ou à des cétènes.

Plus spécifiquement on fait appel à des agents acylant de formule générale:

$$R' - CO - X \qquad (III)$$

dans laquelle:

- R' a la signification donnée ci-avant,
- X représente un groupe hydroxyle, un atome d'halogène ou un reste de formule–O–CO–R'.

Parmi les agents acylant de formule (III) on peut citer: l'acide acétique, l'acide propionique, l'acide butyrique, l'acide isobutyrique, l'acide méthyl-2 butyrique, l'acide méthyl-3 butyrique, l'acide hexanoïque, l'acide diméthyl-3,3 butyrique, l'acide heptanoïque, l'acide dodecaneoïque, l'acide laurique, l'acide stéarique, l'acide cyclohexylacétique, l'acide crotonique, les chlorures, bromures et fluorures d'acétyle, de propionyle, de butyryle, d'isobutyryle,de méthyl-2 butyryle, de méthyl-3 butyryle, d'hexanoyle,

d'heptanoyle, de dodecanoyle, de lauroyle, de stéaroyle, de cyclohexylacétyle, de crotonyle, de chloroacétyle, de bromoacétyle, de chloro-3 propionyle, d'alpha-bromobutyryle, de (p-chlorophényl)-3 propionyle, de benzoyle, de p-toluyle, de m-chlorobenzoyle, de méthoxycarbonyl-4 benzoyle, de p-nitrobenzoyle, de p-anisoyle, d'alpha-naphtoyle; les anhydrides des acides acétique, propionique, butyrique, isobutyrique, méthyl-2 butyrique, méthyl-3 butyrique, hexanoïque, diméthyl-3,3 butyrique, heptanoïque, dodecaneoïque, laurique, stéarique, cyclohexylacétique, crotonique, benzoïque, toluique, p-chlorobenzoïque, chloroacétique, chloro-3 propionique, p-nitrobenzoïque.

On peut également utiliser comme agents acylant des cétènes de formule générale :

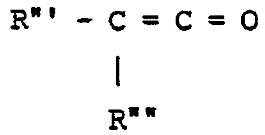

(IV)

dans laquelle R''' et R'''', identiques ou différents, représentent un atome d'hydrogène ou un radical hydrocarboné ayant la signification donnée pour R'. De préférence R''' et R'''' représentent un atome d'hydrogène, un radical alkyle inférieur, ou un radical phényle. Comme exemple de cétènes on peut citer le cétène, le méthylcétène, l'éthylcétène, le n-propylcétène, l'isopropylcétène, le n-butylcétène, le diméthylcétène, l-éthylméthylcétène, le phénylcétène, le méthylphénylcétène.

Parmi les différents agents précités on fait appel de préférence aux halogénures et anhydrides d'acides.

Le trifluorure de bore présente l'avantage par rapport à tous les catalyseurs utilisés dans l'art antérieur de pouvoir être aisément récupéré en fin de réaction par simple distillation en raison de son bas point d'ébullition. Comme l'acide fluorhydrique présente également le même avantage le recours à l'emploi conjoint d'acide fluorhydrique et de trifluorure de bore assure, en plus de taux de conversion et de rendements élevés des biphényles, une grande simplicité de mise en oeuvre de l'invention et la possibilité de récupérer facilement en fin de réaction un mélange $HF/BF_3$ réutilisable pour une nouvelle opération.

L'acide fluorhydrique constitue pour l'essentiel le milieu de réaction. La quantité d'acide fluorhydrique mise en oeuvre varie suivant les conditions de la réaction et la nature des réactifs et plus particulièrement en fonction de leur miscibilité avec HF liquide. En général le rapport pondéral entre l'acide fluorhydrique et le biphényle est compris dans un intervalle d'environ 1 à environ 50. On pourrait naturellement sortir de ces limites sans sortir pour autant du cadre de l'invention. Un rapport pondéral HF/biphényle compris entre 2 et 20 convient bien.

L'emploi d'acide fluorhydrique comme milieu réactionnel n'exclut pas le recours à un tiers solvant pour faciliter la mise en oeuvre du procédé selon l'invention. Ainsi, lorsqu'un des réactifs est solide il peut être avantageux de le mettre en solution dans un solvant inerte avant de l'introduire dans la zone réactionnelle. Dans ce cas on fait appel aux solvants généralement utilisés dans les réactions de Friedel-Crafts tels que ceux cités par G.A. OLAH loc. cit. volume III pages 4 à 5. On peut par exemple utiliser des solvants inertes tels que le sulfure de carbone, le tétrachlorure de carbone, le chlorure de méthylène, le nitrobenzène.

La quantité de trifluorure de bore utilisée peut varier dans de larges limites car il est facilement recyclable, néanmoins d'un point de vue technique on préfère opérer dans une pression absolue de trifluorure de bore comprise entre 5 et 50 bars.

La quantité d'agent acylant exprimée en moles par mole de biphényle peut varier dans de larges limites. Il est préférable que la quantité d'agent acylant soit d'environ la quantité stoechiométrique nécessaire pour introduire deux groupes acyle. Il est préférable d'opérer en présence d'un excès d'agent acylant qui peut être récupéré pour une nouvelle opération. D'une manière générale la quantité d'agent acylant peut être comprise entre 2 et 2,5 moles par mole de biphényle.

La température à laquelle on met en oeuvre le procédé selon l'invention peut varier dans de larges limites. Bien que la température de réaction puisse atteindre 100°C, des températures inférieures ou égales à 80°C sont en général suffisantes pour obtenir des taux de transformation complets du substrat et des rendements quantitatifs. De façon générale la température peut varier d'environ + 50°C à environ 80°C.

La pression à laquelle on conduit le présent procédé est en général au moins égale à celle nécessaire pour maintenir l'acide fluorhydrique à l'état liquide à la température choisie.

D'un point de vue pratique le procédé selon l'invention peut être mis en oeuvre selon les cas dans un appareillage résistant à la pression ou dans un appareillage fait pour travailler à pression normale en polyéthylène par exemple. L'ordre d'introduction des réactifs n'est pas critique. Lorsque la réaction est terminée la masse réactionnelle est traitée pour récupérer le diacyl-4-4'-biphényle obtenu. Selon le cas on peut soumettre la masse réactionnelle à une hydrolyse par de l'eau glacée puis récupérer le produit d'acylation après séparation de la phase organique ou bien procéder à la distillation de l'acide fluorhydrique et le cas échéant du catalyseur. L'acylbiphényle est ensuite récupéré à partir du résidu organique.

Le procédé selon l'invention se prête particulièrement bien à une mise en oeuvre continue.

Les exemples suivants illustrent l'invention et montrent comment elle peut être mise en pratique.

## EXEMPLE 1 COMPARATIF

Dans un réacteur en acier inoxydable de 50 ml équipé d'un système d'agitation et d'un dispositif de refroidissement ou de chauffage, on charge 4,63 g (0,03 mole) de biphényle en solution dans 10 g de chlorure de méthylène. On refroidit le contenu du réacteur à 0°C puis charge 20 g d'acide fluorhydrique anhydre et 3,4 g (0,033 mole) d'anhydride acétique. On ferme l'appareil puis établit une pression de $BF_3$ de 5 bars (soit 7g).

On chauffe à 20°C et maintient cette température pendant 4 heures.

On soutire ensuite la masse réactionnelle sur 100 g de glace puis rince l'appareil 3 fois avec 30 ml de chlorure de méthylène. La phase aqueuse est neutralisée par une solution saturée de carbonate de potassium, puis extraite avec 2 fois 50 ml de chlorure de méthylène. Le solvant est distillé. On obtient 5,9 g de cristaux blancs constitués d'acétyl-4 biphényle pur dosé et identifié par chromotagraphie en phase gazeuse. Le point de fusion du produit obtenu déterminé au banc de KOFLER est de 116°C. Le spectre RMN de produit obtenu est conforme à celui de l'acétyl-4 biphényle. Le rendement en acétyl-4 biphényle est de 100 %.

L'anaylse chromatographique et la RMN montrent que la totalité du biphényle a été transformée.

Le taux de conversion du biphényle est de 100 % et le rendement réel en diacétyl-4-4'-biphényle est de 0 %.

EXEMPLE 2

Dans un réacteur de 50 ml en acier inoxydable, équipé d'un système d'agitation et d'un dispositif de refroidissement ou de chauffage, on charge 4,63 g (0,03 mole) de biphényle. On refroidit à 0° puis charge 20 g d'acide fluorhydrique anhydre et 6,8 g (0,066 mole) d'anhydride acétique. On établit une pression de $BF_3$ de 5 bars (soit environ 7 g).

On chauffe à 50°C et maintient cette température pendant 4 h.

On traite selon l'exemple 1 et on obtient un mélange de 1,2 g d'acétyl-4 biphényle et 5,5 g de diacétyl-4-4' biphényle, soit un rendement de 76 %.

EXEMPLE 3

On reproduit l'exemple 2 en mettant en oeuvre
20 ml de $CH_2$ $Cl_2$
4,63 g (0,03 mole) de biphényle
6,8 g (0,066 mole) d'anhydride acétique
On établit une pression de $BF_3$ à 5 bars
On chauffe à 50°C pendant 5 heures
·On traite selon l'exemple 1 et on obtient 5,86 g d'acétylbiphényle soit un rendement de 100 % en acétylbiphényl.

**Revendications**

1°) Procédé de préparation de diacyl 4-4'-biphényle caractérisé en ce qu'on met en contact à une température supérieure ou égale à 50°C le biphényle et un agent acylant dans l'acide fluorhydrique liquide en présence de trifluorure de bore en quantité telle que la pression absolue de trifluorure de bore dans l'enceinte réactionnelle soit d'au moins un bar.

2°) Procédé selon la revendication 1, caractérisé en ce que l'agent acylant est pris dans le groupe des composés de formules générales :

$$R' - CO - X \qquad (III)$$

dans laquelle :
- R' représente un reste hydrocarboné ayant de 1 à 30 atomes de carbone éventuellement substitués par un ou plusieurs atomes d'halogènes et/ou groupes fonctionnels,
- X représente un groupe hydroxyle, un atome d'halogène ou un reste de formule - O - CO - R',

$$R''' - \underset{\displaystyle R''''}{\underset{|}{C}} = C = O \qquad (IV)$$

dans laquelle R''' et R'''', identiques ou différents, représentent un atome d'hydrogène ou un radical hydrocarboné ayant la signification donnée pour R'.

3°) Procédé selon la revendication 2, caractérisé en ce que l'on utilise l'anhydride acétique comme agent acylant.

4°) Procédé selon l'une quelconque des revendications 1 à 3, caractérisé en ce que la température de la réaction peut prendre toute valeur de l'intervalle de + 50°C à 100°C.

5°) Procédé selon l'une quelconque des revendications 1 à 6, caractérisé en ce que la quantité d'agent acylant exprimée en moles par mole de biphényle peut varier dans un intervalle de 2 à 2,5.

**Patentansprüche**

1. Verfahren zur Herstellung von 4-4'-Diacylbiphenyl, dadurch gekennzeichnet, daß man bei einer Temperatur oberhalb von oder gleich 50°C Biphenyl und ein Acylierungsagens in flüssiger Fluorwasserstoffsäure in Gegenwart von Bortrifluorid in einer solchen Menge in Kontakt bringt, daß der absolute Druck an Bortrifluorid im Reaktor wenigstens 1 bar beträgt.

2. Verfahren nach Anspruch 1, dadurch gekennzeichnet, daß das Acylierungsagens aus der Gruppe der Verbindungen der allgemeinen Formel:

$$R' - CO - X \qquad (III)$$

ausgewählt wird, in der:
– R' einen Kohlenwasserstoffrest mit 1 bis 30 Kohlenstoffatomen darstellt, der eventuell substituiert ist mit ein oder mehreren Halogenatomen und/oder fuktionellen Gruppen,
– X einen Hydroxylrest, ein Halogenatom oder ein Rest der Formel –O–CO–R', darstellt,
oder

$$R''' - \underset{\displaystyle R''''}{\underset{|}{C}} = C = O \qquad (IV)$$

in der R''' und R'''' identisch oder verschieden sind und jeweils ein Wasserstoffatom oder einen Kohlenwasserstoffrest mit der für R' angegebenen Bedeutung darstellen.

3. Verfahren nach Anspruch 2, dadurch gekennzeichnet, daß man Acetanhydrid als Acylierungsagens verwendet.

4. Verfahren nach einem der Ansprüche 1 bis 3, dadurch gekennzeichnet, daß die Reaktionstemperatur einen Wert im Bereich von +50°C bis 100°C einnimmt.

5. Verfahren nach einem der Ansprüche 1 bis 4, dadurch gekennzeichnet, daß die Menge des Acylierungsagens ausgedrückt in Molen pro Mol an Biphenyl im Bereich von 2 bis 2.5 variiert.

## Claims

1. Process for preparing 4,4'-diacylbiphenyl characterized in that biphenyl is brought into contact with an acylating agent in liquid hydrofluoric acid at a temperature greater than or equal to 50°C, in the presence of boron trifluoride in such an amount that the absolute pressure of boron trifluoride in the reaction chamber is at least one bar.

2. Process according to Claim 1, characterized in that the acylating agent is selected from the group consisting of compounds of general formulae:

$$R' - CO - X \qquad (III)$$

in which

R' denotes a hydrocarbon residue having from 1 to 30 carbon atoms and optionally substituted with one or more halogen atoms and/or functional groups.

X denotes a hydroxyl group, a halogen atom or a residue of formula –O–CO–R',

$$R''' - \underset{\displaystyle R''''}{\underset{|}{C}} = C = O \qquad (IV)$$

in which R''' and R'''', which may be identical or different, denote a hydrogen atom or a hydrocarbon radical having the meaning given for R'.

3. Process according to Claim 2, characterized in that acetic anhydride is used as acylating agent.

4. Process according to any one of Claims 1 to 3, characterized in that the reaction temperature can assume any value in the range from +50°C to 100°C.

5. Process according to any one of Claims 1 to 4, characterized in that the amount of acylating agent expressed in moles per mole of biphenyl can vary within the range from 2 to 2.5.